# EUROPEAN PATENT SPECIFICATION

(11) **EP 1 468 622 B1**
(45) Date of publication and mention of the grant of the patent: **05.11.2008**
(21) Application number: 04251742.5
(22) Date of filing: 25.03.2004
(51) Int. Cl.: A44B 19/00, A44B 19/30, A44B 19/36, B60R 21/20

(54) **Zip fastener**
Reissverschluss
Fermeture à glissière

(30) Priority: 31.03.2003 GB 0307401
(43) Date of publication of application: 20.10.2004
(73) Proprietor: YKK Corporation, Chiyoda-ku, Tokyo (JP)
(72) Inventor: Meirion, Williams, YKK Europe Ltd., Runcorn WA7 3BW Cheshire (GB); Shunji, Akashi, YKK Europe Ltd., Runcorn WA7 3BW Cheshire (GB); Shigeyoshi, Takasawa, YKK Europe Ltd., Runcorn WA7 3BW Cheshire (GB)
(74) Representative: Luckhurst, Anthony Henry William

(56) References cited:
- WO-A-00/35719
- CA-A- 2 207 022
- GB-A- 527 825
- GB-A- 678 755
- US-A- 2 701 903
- US-A- 3 953 912
- US-A- 5 588 671
- US-B1- 6 705 642

## Description

The present invention relates to fasteners, and in particular to a fastener for closing an airbag cover.

Cars are often provided with airbags, for use as a safety device in case of a collision. An airbag is provided at the front of the car, e.g. on the central part of the steering wheel. If a collision occurs, a sensor detects the collision, and the airbag is immediately inflated. This will cushion a person who is thrown forwards from the car seat.

It is becoming more common for cars to be provided with side airbags as well as front airbags, to give increased protection in the event of a side-on collision. The side airbags inflate into a space between the car seat and the door of the car, cushioning an occupant of the seat against an impact with the side of the car. A common place to install side airbags is inside the back of the car seat. When a collision occurs, the airbag inflates, and pushes through an opening, such as a slit, in the side of the car seat. The emerging airbag expands along the side of the car, to protect the driver from hitting the car door.

The opening on the car seat, through which the airbag emerges, should be kept closed in normal use, but should open as the airbag inflates. It is known to keep the airbag opening closed by having it sewn up with yarn. When the airbag inflates, the pressure against the yarn causes the yarn to break, allowing the airbag to emerge from the car seat, and protect an occupant of the car.

However, it is not a straightforward process to sew up the airbag opening. The opening must be sewn to controlled criteria, such as yarn type, number and type of stitches, tension, etc. to ensure the yarn breaks when the airbag inflates, but also to ensure the yarn does not break during normal use of the vehicle.

US-A-5588671 describes a car seat having a resealable tear seam for deployment of a side impact air bag. WO 0035719 describes a zipper like closure with a break line at one end, for closing an airbag cover. GB-A-678755 describes containers for inflatables and parachutes, in which a zip fastener can be burst apart to open the container.

CA-A-2207022 describes a zip fastener having an end stop mid way along its length the end stop parts being coupled by movement laterally of the fastener tapes.

It is sometimes necessary to gain access to an airbag for routine checking or maintenance, in which case the yam must be removed and the opening re-sewn to the required criteria after the maintenance is completed.

We aim to provide a closure for an airbag opening which overcomes some of the problems mentioned above.

According to the present invention, we provide an air bag cover, as set forth in claim 1.

A zip fastener provides a mechanism for easily closing an opening. It is known that zip fasteners can be opened (or broken) at a point along the row of coupling elements if sufficient force is applied to the fastener to separate the coupling elements of the fastener. It is difficult to provide a fastener in which the coupling elements will reliably disengage in this way when a predetermined force is applied. However, the engaging coupling elements can be readily peeled apart once a break is made, or from an end of the row of elements if, for example, the slider inadvertently moves past the upper end stops.

Thus, we provide a zip fastener having a pair of tapes which are joined by rows of coupling elements mounted on the respective tapes, wherein a point of weakness is provided at a predetermined position along the fastener so that the coupling elements will separate at the point of weakness when a predetermined force is applied to the fastener. More than one point of weakness may be provided along the fastener.

Preferably, a frangible connection or a releasable connection is provided at the point of weakness in order to retain the adjacent elements in engagement until the connection is broken or released.

In another embodiment, a slider provides the point of weakness. Pressure transverse to the plane of the fastener at the slider will cause the tapes to bulge outwards and urge the slider back along the row of elements, opening the zip fastener. Two sliders can be provided, the sliders closing the fastener from opposite directions, and meeting at an intermediate position to close the fastener. The sliders may be coupled together by a frangible or releasable connection.

Preferably, the or a point of weakness is provided in a central region, when measured from the ends of the opening provided by the opened fastener. Preferably a point of weakness is provided in a central half of the fastener, in a region between one quarter and three quarters of the length of the fastener. More preferably, a point of weakness is provided substantially mid-way along the fastener, i.e. at half the length of the fastener.

A force for releasing the holding means will come from the pressure of the airbag against the fastener. However, although this pressure will primarily be in a direction perpendicular to the plane of the fastener, for example the side of the seat, the fastener is also stretched within the plane of the stringer tapes. The force (or displacement) from the airbag, perpendicular to the side of the seat and/or the stretching force, may be utilised to open the fastener.

As mentioned above, coupling elements are more easily separated by peeling apart from open region of the fastener than within a connected region of the fastener. The tapes may be joined at ends of the first and second areas furthest from the opening point, to prevent the fastener from opening at those ends.

The holding means for holding the tapes together may take a variety of forms.

One form comprises a pair of sliders which are mounted onto the first and second areas of coupling elements respectively, to open the coupling elements when the sliders are moved away from one another, and to close the coupling elements when moved towards one another. As well known in the art, the coupling elements at the leading end of a slider are separated within the slider body. Hence, with appropriate slider geometry, the sliders can be urged back along the tapes, opening the fastener, by a force which causes the tape to bulge in the region of the sliders. Preferably a stop is provided to position the sliders when the fastener is closed, the sliders providing a point of weakness where the force of the airbag will cause the fastener to start to open.

The two sliders may be provided without a puller, or with a removable puller for removal after the fastener has been installed and fastened, so that the slider are less likely to be accidentally moved or interfered with while the fastener is installed on a car seat.

A link may be provided for linking two sliders together, the link being broken or separated by the force of the exploding air bag, for example. The link may be cooperating locking parts provided on the slider bodies or provided on the slider pull tabs, for example.

The link may directly connect the first slider to the second slider, co-operating formations on the two sliders holding them together. Thus, the link is separated or broken in order to allow the sliders to move apart to open the fastener.

An intermediate linking element may be provided, the linking element being connected to the two sliders.

The force required to separate the two sliders will be dependent on factors such as the chosen size and type of linking means, and the ease of moving the slider along the coupling elements on the stringer tape, etc.

In another embodiment, the holding means may comprise a link which joins the stringer tapes when the fastener is closed. The link may be a frangible element, needing replacement after it has been broken a single time. Alternatively, the link may be reconnectable, such that if the link is opened, it fastener may subsequently be closed again by closing the coupling elements in the first and second areas and re-closing the link. The link may be made of a plastics material.

A frangible link may be formed by welding, fusing or melting an area of the stringer tapes to join the two stringer tapes, or by attaching a piece of material across the two stringer tapes to bridge them. The force required to break the link may be determined by the material, by the welding or joining process used, by an area over which the join or weld extends, and/or by providing a weakened region, etc.

A replaceable disposable link which is breakable when a large enough force is applied to it, such as a clasp, clip, or breakable moulded element, may be provided. The disposable link may be directly connected to both the stringer tapes. In another embodiment it may be connected to the stringer tape via a piece of tape. This tape may be welded, glued or otherwise fixed onto the stringer tape. The disposable link may comprise a single breakable part, and a line of weakness may be provided on the breakable part, such as a thin area, along which the link will break under tension.

The disposable link may comprise two parts which are connected together, but which separate when a sufficient force is applied.

The airbag expansion force, i.e. the force at which the fastener should open, is expected to be around 400 Newtons, although this may vary with the model of car used and the size of airbag used. The holding means must be chosen such that the fastener will open under the pressure of the exploding air bag, but will not during normal use of the vehicle. Preferably the force required is less than 400 N but greater than 300 N.

Preferably the length of the fastener is around 50cm.

TGaps are formed along the rows of coupling elements to provide the first and second areas. The gap may be located in the centre of the fastener, or in an off-centre position.

The fastener may be arranged to open at more than one point along its length. A plurality of gaps may be provided, dividing the rows into more than two areas. A link may be provided in each gap.

The coupling elements may be helically wound coil elements or may be discrete interlocking teeth elements of metal or moulded plastics as well known in the art.

The fastener may be reusable or may be designed for a single use.

The coupling elements are mounted on a longitudinal marginal edge of the stringer tape. Where a slider is provided, it may be moved along the stringer tapes to bring the fastener element rows into or out of engagement with each other.

After the fastener has opened, and the airbag has inflated, the airbag may subsequently be removed or deflated. A replacement airbag may be put inside the opening in the car seat, and the fastener may be closed, to make it ready for use again. If the fastener comprises a slider, the slider may be used to close the coupling elements. However, if the fastener does not comprise a slider, a special tool may be used to close the coupling elements. A known example of such a tool is a jig with a part for pressing the coupling elements together, and a handle for moving the jig along the row of coupling elements.

Preferred embodiments of the present invention will now be described by way of example only and with reference to the accompanying drawings, in which:
Figure 1A shows a top view of a first embodiment of a fastener for use in the airbag cover of the invention, and figure 1B shows a perspective view of the link of the first embodiment;
Figure 2 shows a perspective view of a car seat with the fastener of the first embodiment on the side of the seat;
Figure 3 shows a top view of a second embodiment of the fastener in which a tape or a weld link is provided to link the stringer tapes together;
Figure 4A shows a top view of a third embodiment of the fastener, in which the link is provided by a clip. Figure 4B shows a top view of the clip when open, and figure 4C shows a cross sectional view of the clip;
Figures 5A-C show a perspective view and two cross sectional views of a fourth embodiment of the fastener in which the link is provided by a two part link with a pushing pin to allow separation when a force is applied to the pin;
Figure 6 shows a fifth embodiment of the fastener in which a pair of sliders is provided;
Figure 7 shows a top view of a sixth embodiment of the fastener in which a pair of linkable sliders are provided;
Figure 8 shows a top view of a seventh embodiment of the fastener in which a pair of sliders with a separate linking element is provided to connect the sliders;
Figures 9A-D show perspective views of a pair of sliders and pulltabs according to an eighth embodiment of the fastener, and
Figure 10 shows a side view of a jig for closing the coupling elements on a fastener without a pair of sliders.

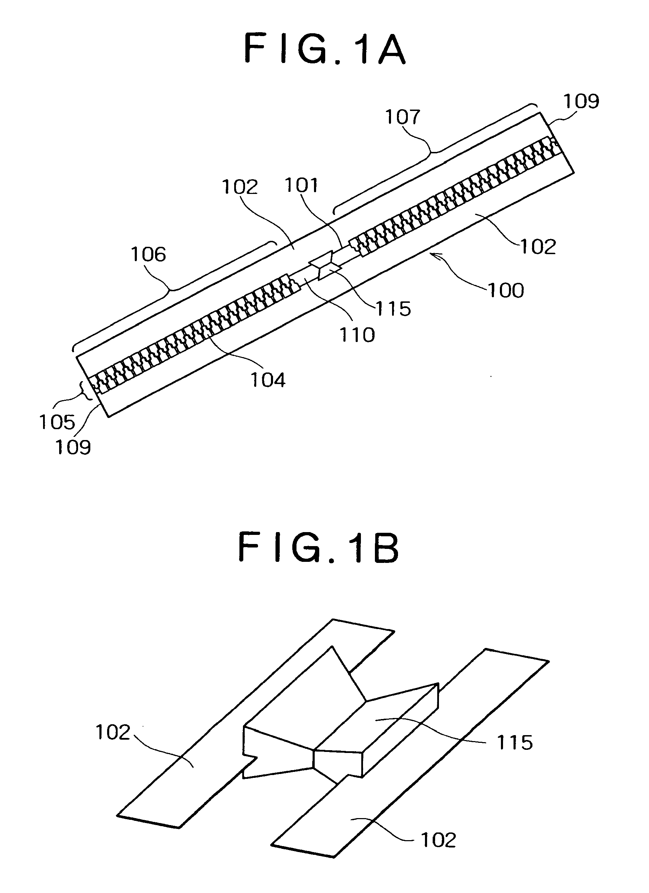

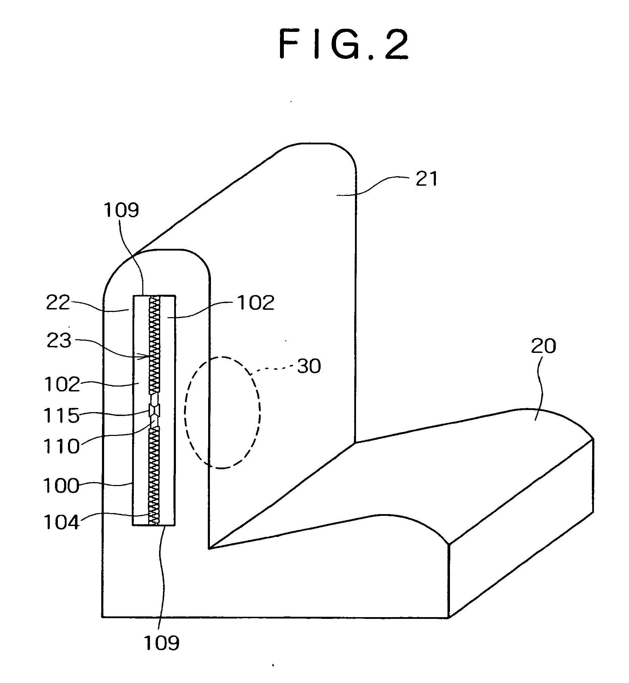

Figure 1A shows a first embodiment of a fastener for use in an airbag cover of the invention, with the fastener closed, and figure 2 shows the fastener 100 of the first embodiment in position on a car seat 20.

The fastener 100 comprises two stringer tapes 102, with a plurality of coupling elements 104 mounted on the inner longitudinal marginal edges 101 of the stringer tapes 102. The coupling elements 104 interlock to close the fastener and connect the stringer tapes 102 together, as well known in the art. With the fastener closed, the coupling elements 104 are engaged in a first area 106 and a second area 107. The region between the first and second areas does not have coupling elements 104 attached, but instead, a gap 110 is provided between the first and second areas. The gap 110 is located in the centre of the fastener. A link 115 is provided in the centre of the gap, joining the two stringer tapes 102 together at this point to prevent the coupling elements peeling apart. The ends 109 of the fastener 100 are fixed together by a link (not shown) or by sewing to the seat cover

Figure 1B shows a perspective view of the link 115. The link 115 may be formed of plastics by insert moulding on to the opposed edges 101 of the tapes 102, a waisted region being formed, at which the link will break when subjected to a predetermined force. In another embodiment, halves of the link may be formed on respective tape edges, and then joined at the waisted region by ultrasonic welding, for example.

As shown in figure 2, the car seat 20 has a frame shaped like the seat and a back cover 21 covering the frame inside which back cover an airbag 30 has been installed. The side 22 of the back cover 21 of the car seat 20 has an opening 23 running vertically along it, the opening being about 500mm in length. A fastener 100 according to the first embodiment of the invention is sewn into the opening in the car seat cover. When a collision occurs, the airbag 30 will explode or expand rapidly and push against the fastener 100. The gap 110 and the link 115 are positioned at a location which will be impacted by the exploding air bag. As the airbag 30 expands, the link 115 will break and the coupling elements 104 in the first and second areas will subsequently peel apart, thus opening the entire fastener to allow the air bag to expand through the opening 23.

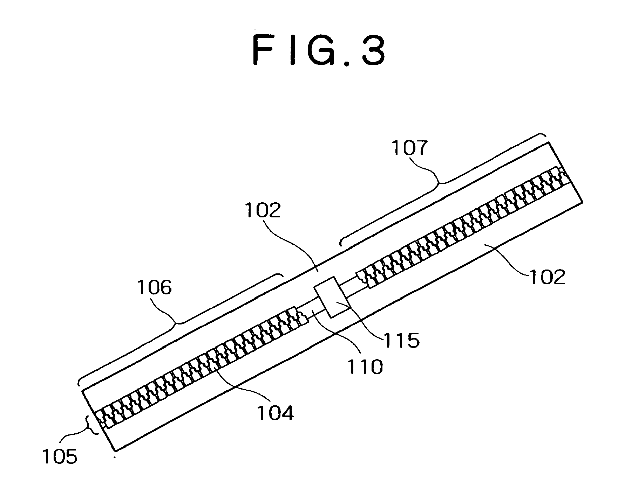

Figure 3 shows a second embodiment of the fastener, again showing the fastener while it is closed. A pair of stringer tapes 102 are provided with a plurality of coupling elements 104 attached to their edge, to allow the stringer tapes 102 to be connected. A gap 110 is provided between a first and second areas 106, 107 of the coupling elements.

A link 115 is provided. In this embodiment, the link comprises a weld or a tape link. The weld link may be formed by partially melting adjacent areas of the two stringer tapes, for example with ultrasonic welding, in order to join the stringer tapes. This is particularly suitable for helical filament type fastener elements where the heads of the coupling elements are quite short and so the tape edges tend to be quite close together. A tape link may be formed by attaching a piece of tape, such as taffeta tape, by gluing, sewing or welding to both stringer tapes to bridge the gap between the stringer tapes. When force is applied by an airbag, the weld or tape link will break, and the fastener will then open.

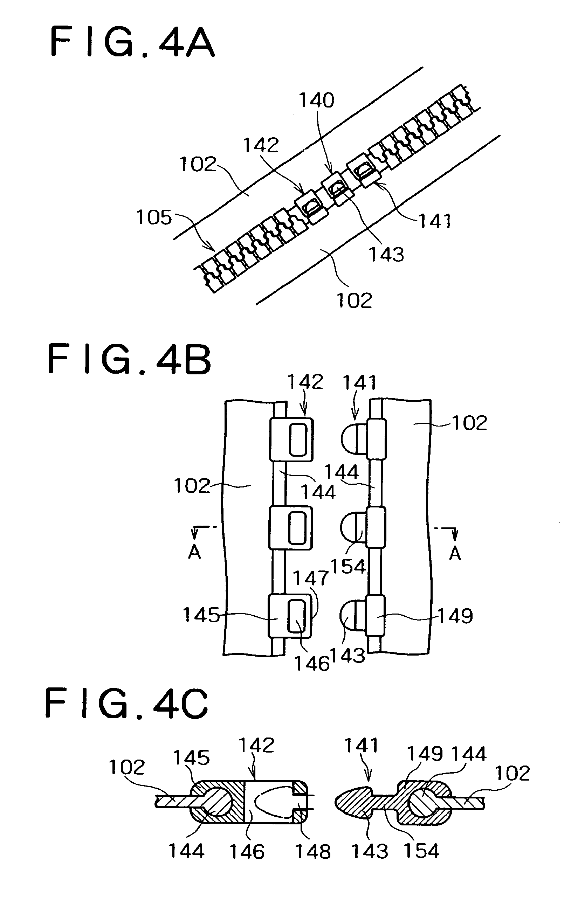

Figure 4A shows a third embodiment of the fastener in which the link 115 comprises a clip 140. The clip 140 comprises a first, male part 141 and a second, female part 142 which are connected to the first and second stringer tapes respectively. The first 141 and second 142 parts of the clip 140 engage together to close the clip 140 and join the stringer tapes. Male part 141 has an arrow shaped engaging head 143 which is received in the female part 142 to hold the two parts 141, 142 together. Such clip designs are well known. The clip 140 may be welded directly to the tape, or may be welded to another piece of tape which is attached to the stringer tapes. The clip 140 may be reusable, opening under a predetermined force but being reconnectable.

Figures 4B and 4C show more detailed views of the structure of the clip 140 of figure 4A. Figure 4B is a top view, showing three open clips 140 attached to stringer tapes, and figure 4C is a cross sectional view of one of the clips 140, taken along line A-A of figure 4B. A core portion 144 is formed on the edge of each stringer tape 102. Both the female 142 and male 141 parts of the clip 140 have a leg portion 145, 149 for sandwiching the core portions 144 from above and below to hold the clip parts 141, 142 onto the stringer tapes 102. The female part 142 of the clip has a cavity portion 146 which is long in a longitudinal direction of the stringer tape 102 and which vertically penetrates a front face of the leg portion 145. A front wall 147 at the end of the cavity portion 146 is defined with a rectangular engaging hole 148 which is long in the longitudinal direction of the tape such that the engaging hole 148 communicates with the cavity portion 146 and the engaging head 143 of the male part 141 can be inserted into and engaged with the engaging hole 148.

The male part 141 has a thin neck portion 154 projecting from a front face of the leg portion 149 and having a width smaller than that of the leg portion 149 and substantially equal to that of the engaging hole 146. The engaging head 143 is mounted to an end of the neck portion 154, and is arc-shaped, having the same width as that of the neck portion 154 and being triangular in cross section such that the engaging head 143 can receive the front wall 147 of the female part 142.

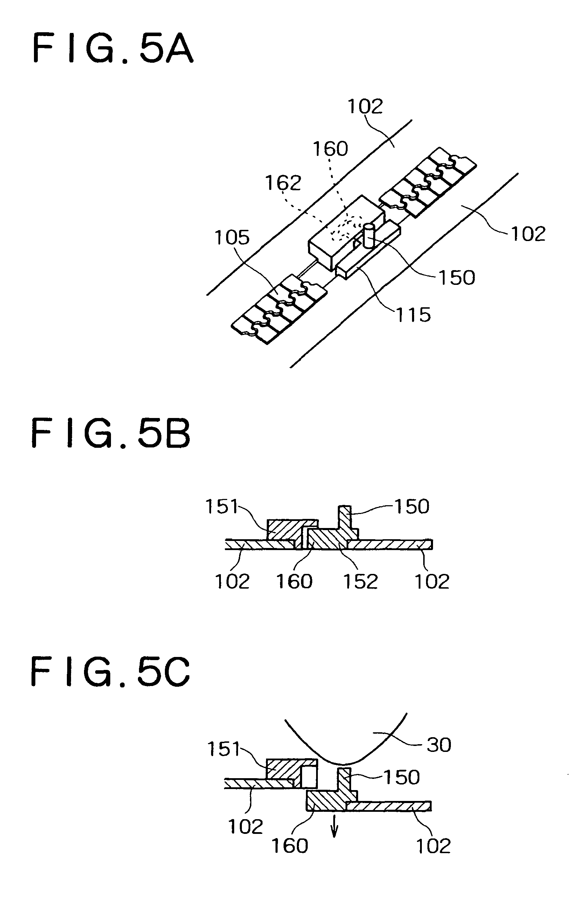

Figures 5A-C show a fourth embodiment of the fastener, comprising a fastener 100 which opens due to direct displacement of a part of a link by the airbag. The fastener comprises a two-part link element 115 which opens in a direction perpendicular to the stringer tapes. A projection 150 is provided on one part 152 of the link element 115 and faces the air bag. The projection 150 is positioned over the airbag, in a position in which the airbag will give the greatest displacement. In this embodiment, the projection 150 is a blunt ended pin.

Figure 5A shows a perspective view of the link element with the projection. Figure 5B shows a cross-sectional view of the link element when it is in a closed position, and Figure 5C shows a cross-sectional view of the link element when it is in an open position.

The link element 115 comprises first 152 and second 151 parts, with the projection 150 provided on the first part 152, and a linking part also being provided on the first part 152. The linking part 160 is in the from of a tongue which is received in a complementary shaped blind recess 162 in the second part 151 of the link element, preventing separation of the parts 151, 152 in the plane of the tapes 102.. The linking part 160 is displaced from the recess 162 when pressure is applied to the pin 150 by the exploding air bag, in a direction perpendicular to the stringer tapes 102.

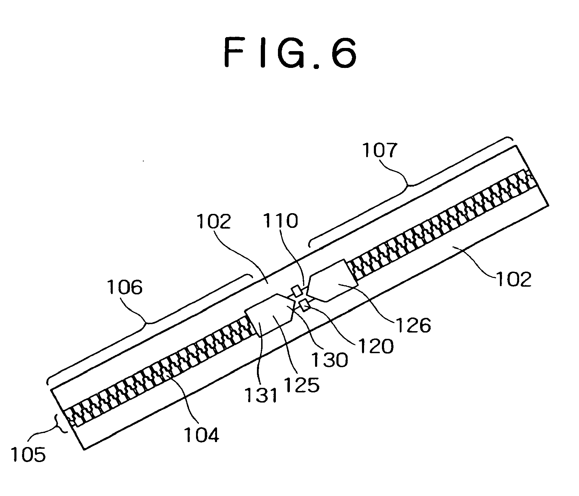

Figure 6 shows a fifth embodiment of the fastener in which the fastener is again shown in a closed state. As in the previous embodiments, a pair of stringer tapes 102 are provided, each stringer tape having a first area 106 and a second area 107 with coupling elements mounted along an inner edge 101 of the stringer tape 102. A stop 120 is provided in a gap 110 in the rows of fastener elements, to prevent movement of a slider 125, 126 too far along the tapes, thus ensuring that the sliders meet at the required position for engagement by the exploding air bag. Such stops are well known in the art and may be provided for example by attaching clips to the tapes.

Instead of a link 115 etc. being provided to connect the stringer tapes in the gap 110, the fastener is held closed by the pair of sliders 125, 126 mounted on the fastener chain 105. Each slider has a front end 130 and a back end 131. As well known in the art, forward movement of the slider along the stringer tapes closes the fastener, and movement backwards opens the fastener.

When the air bag expands, the pressure of the bag will cause the fastener 100 to bulge outwards, tending to separate the tapes 102 at the gap 110 and causing the sliders to move back along the tapes, opening the fastener.

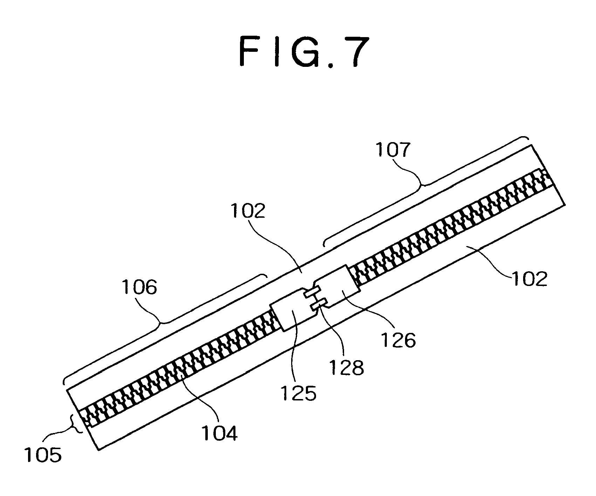

Figure 7 shows a sixth embodiment of the fastener. This is similar to the fifth embodiment, except that in this case the two sliders 125, 126 are connected to each another. In this embodiment each slider comprises a female element and a male element. The female element of one slider is connectable with the male element of the other slider, and vice versa, to allow the two sliders to connect together. When a force is applied from the exploding airbag, the two sliders are pushed apart, thus disengaging the link between them. The strength of the link between the sliders can be controlled, thus providing more control over the force required to open the fastener.

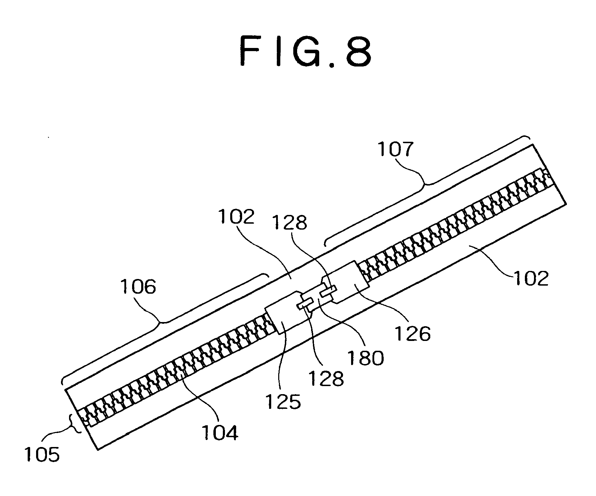

Figure 8 shows a seventh embodiment of the fastener. This is similar to the sixth embodiment, except that instead of the two sliders 125, 126 linking directly to one another, each of the sliders links to a common link element 180. The length of the link element 180 may be varied to modify the minimum force required to open the link. The type of connection used between the link element and the first and second sliders will also influence the strength of the link.

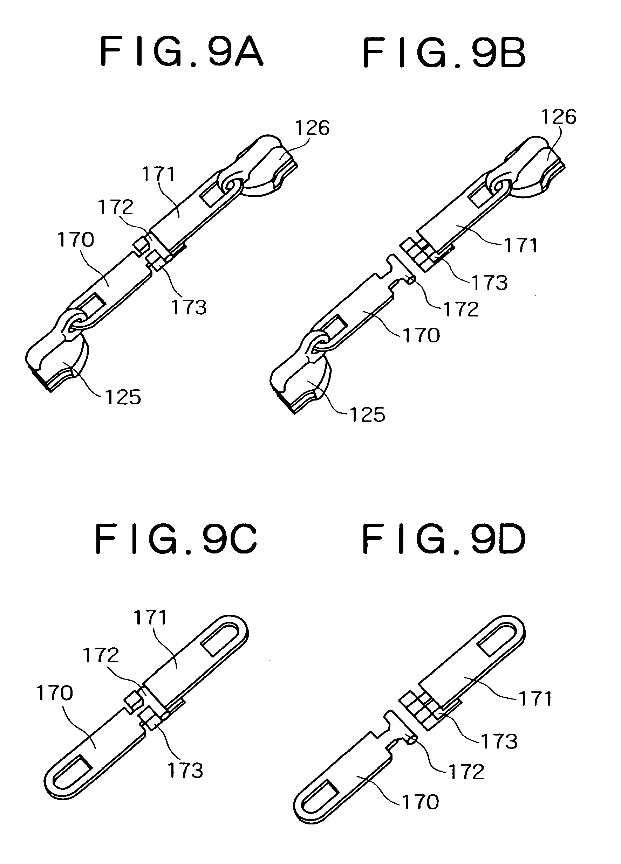

Figures 9A-D show perspective views of a pair of sliders and pulltabs forming an eighth embodiment of the fastener. The eighth embodiment is similar to the sixth embodiment, except that each slider 125, 126 is provided with a pulltab 170, 171, and the two pulltabs 170, 171 are provided with means to connect to each other. Figure 9A shows a pair of sliders 125, 126 and pulltabs 170, 171 when connected together, and figure 9B shows a pair of sliders 125, 126 and pulltabs 170, 171 when disconnected. Figure 9C shows an enlarged view of the two pulltabs 170, 171 when connected together, and figure 9D shows an enlarged view of the two pulltabs 170, 171 when disconnected.

One pulltab 170 is provided with a male connector element 172, and the other pulltab 171 is provided with a female connector element 173. The male connector element 172 is a snap-fit in the recess provided by the female connector element 173. When the airbag expands, the force of the airbag disengages the two connector elements 172, 173, and allows the fastener to open. The coupling parts 172 and 173 may deform on being separated, in which case the pull tabs could be removed and fresh pull tabs attached to the sliders for re-use of the fastener.

The weak point of the zip fastener is formed by the connection of the pull tabs which provide for opening and closing easily as well as providing a controlled breaking or bursting point of the fastener. It is also possible to use a combination pull tab wherein each of the two sliders has the same design. For example, each slider may be provided with both a male connector element and a female connector element. The pulltabs shown in figures 9A-D are solid parts, but alternatively, they could be fabric pulltabs. Fabric pull tabs may be connected with a snap fastening button such as a press stud or a 'linesnap' fastener as marketed by YKK, instead of the connector elements shown in figures 9A-D.

In a further embodiment, instead of being linked by a re-usable link, the two sliders are linked by a solid clip which breaks off with the force of the airbag. This embodiment provides a one-time use. The clip is free to fly off in any direction, however, if preferred, one side of the clip may be permanently fixed to one of the sliders, to prevent the clip from flying off completely.

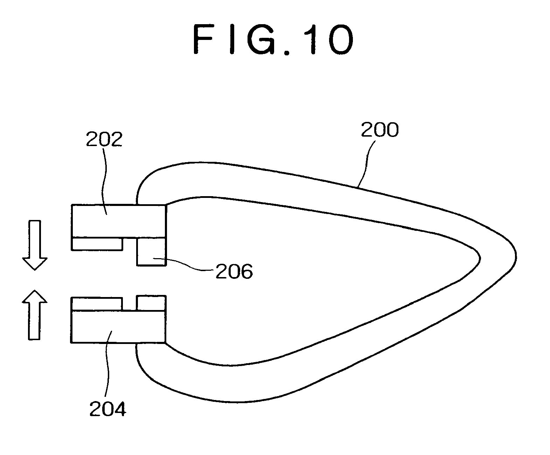

Figure 10 shows a jig which may be used to re-close the coupling elements on the stringer tapes after the fastener has been opened, in the absence of a slider. The jig may be closed around teeth of the fastener element and slid along the length of the stringer tape, closing the coupling elements as it is moved. Such jigs are well known in the art of zip fastener manufacture. To be specific, as shown in Figure 10, the jig is comprised of a curved frame member 200, and a pair of upper and lower wings 202 and 204 mounted on the upper and lower ends, respectively, of the frame member 200. The upper wing 202 has a guide post 206 mounted on its lower side. The upper wing 202 and the lower wing 204 are movable toward and away from each other. When the guide post 206 of the upper wing 202 comes into contact with part of the lower wing 204, the upper wing 202 and the lower wing 204 define therebetween a Y-shaped channel through which rows of coupling elements 104 pass. As the jig is slid longitudinally of the stringer tapes with the coupling element rows passing through the Y-shaped channel, the jig closes the coupling elements, as a slider does.

## Claims

1. An airbag cover having an opening (23) for the release therethrough of an airbag covered by the cover when the airbag inflates, the opening being closed by a fastener (100) comprising first and second stringer tapes (102), each with a row of coupling elements (104) mounted thereon, such that when the fastener is closed, said stringer tapes (102) are connected to each other by means of said coupling elements (104),
holding means (115; 125; 126) for holding said tapes (102) together at a predetermined position, the holding means (115; 125; 126) being released when a force which is greater than a predetermined value is applied to the holding means (115; 125; 126) by the impact of the airbag (30) when the airbag is inflated to allow or cause the coupling elements to disengage, **characterised in that**
each row of coupling elements (104) comprises a first area (106) and a second area (107) where the coupling elements (104) on the first and second tapes. (102) are engaged when the fastener is closed, the coupling elements (104) within the first (106) and second (107) areas disengaging when the airbag is inflated, and
a gap (110) is provided between the first and second areas (106, 107), the holding means (115; 125; 126) being provided at the gap (110) and the first and second areas (106, 107) extending on respective opposite sides of the holding means (115; 125; 126).

2. An airbag cover as claimed in claim 1, wherein said holding means comprises a pair of sliders (125; 126), and a stopper (120) is provided to stop the sliders (125, 126) at the predetermined position.

3. An airbag cover as claimed in claim 2, wherein said holding means comprises linking means to connect said pair of sliders (125; 126) together.

4. An airbag cover as claimed in claim 3, wherein said linking means is an element (180) separate to each slider (125; 126), said linking means having first engaging means for engaging with the first slider (125), and second engaging means for engaging with the second slider (126).

5. An airbag cover as claimed in claim 1, wherein said holding means comprises a link (115) which connects the stringer tapes (102) when the fastener is closed.

6. An airbag cover as claimed in claim 5, wherein said link (115) comprises a connection which is disengaged to open the fastener and re-engaged to re-form the link (115) after it has been disengaged by said force.

7. An airbag cover as claimed in claims 5 or claim 6, wherein said link (115) comprises a female element and a male element, said female element being engaged with said male element to close the link (115).

8. An airbag cover as claimed in claim 5 or claim 6, wherein said link (115) comprises a projection (150) extending away from the surface of the stringer tapes (102), whereby said link (115) is disengageable when said force is applied to said projection (150).

9. An airbag cover as claimed in claim 5, wherein said link (115) comprises a frangible element which is breakable to disengage the link.

10. An airbag cover as claimed in claim 9, wherein said link (115) comprises a fused area of stringer tape.

11. An airbag cover as claimed in claim 9, wherein said link comprises an additional area of tape which is fused onto said stringer tape.

12. An airbag cover as claimed in any one of the previous claims, wherein the force required to release or displace the holding means (115; 125; 126) is less than 400 N, said force being applied to an area of the fastener adjacent to the holding means (115; 125; 126), in a direction substantially perpendicular to the surface of the stringer tape (102) adjacent to the holding means (115; 125; 126).

13. An airbag cover as claimed in any one of the previous claims, wherein the force required to release the holding means (115; 125; 126) is greater than 300 N, said force being applied to an area of the fastener adjacent to the holding means (115; 125; 126), in a direction substantially perpendicular to the plane of the stringer tape (102) adjacent to the holding means (115; 125; 126).

14. An airbag cover as claimed in any one of the preceding claims, said cover being in the form of a cover for a motor vehicle seat (20), said fastener (100) being attached to said cover (21) at said opening (23) to close said opening (23).

15. A seat (20) for a motor vehicle, said seat comprising a frame, a cover (21) as claimed in any one of claims 1 to 13, mounted on said frame, an airbag (30) within said frame, being said fastener (100) being attached to said cover at said opening (23) to close said opening (23) in normal use and to open under the impact of the air bag when the airbag (30) is inflated to allow the airbag (30) to pass through the opening (23).

## Patentansprüche

1. Airbag-Abdeckung mit einer Öffnung (23) für die Freigabe eines von der Abdeckung abgedeckten Airbags durch die Öffnung, wenn der Airbag aufgeblasen wird, wobei die Öffnung von einem Verschluß (100) geschlossen ist, der erste und zweite Verschlußbänder (102) aufweist, jedes mit einer Reihe von derart darauf angebrachten Kopplungselementen (104), daß die Verschlußbänder (102) miteinander mittels der Kopplungselemente (104) verbunden sind, wenn der Verschluß geschlossen ist,
einer Halteeinrichtung (115; 125; 126) zum Zusammenhalten der Bänder (102) an einer vorbestimmten Stelle, wobei die Halteeinrichtung (115; 125; 126) freigegeben wird, wenn durch den Aufprall des Airbags (30) eine Kraft größer als ein vorbestimmter Wert auf die Halteeinrichtung (115; 125; 126) aufgebracht wird, wenn der Airbag aufgeblasen wird, um eine Entkopplung der Kopplungselemente zu erlauben oder zu verursachen, **dadurch gekennzeichnet, daß**
jede Reihe von Kopplungselementen (104) einen ersten Bereich (106) und einen zweiten Bereich (107) aufweist, wo die Kopplungselemente (104) an dem ersten und dem zweiten Band (102) gekoppelt sind, wenn der Verschluß geschlossen ist, wobei die Kopplungselemente (104) innerhalb des ersten (106) und zweiten (107) Bereichs entkoppeln, wenn der Airbag aufgeblasen wird, und
eine Lücke (110) zwischen dem ersten und dem zweiten Bereich (106, 107) vorgesehen ist, wobei die Halteeinrichtung (115; 125; 126) an der Lücke (110) vorgesehen ist und sich der erste und der zweite Bereich (106, 107) an jeweils entgegengesetzten Seiten der Halteeinrichtung (115; 125; 126) erstrecken.

2. Airbag-Abdeckung nach Anspruch 1, bei der die Halteeinrichtung ein Paar von Schiebern (125; 126) aufweist und ein Stopper (120) vorgesehen ist, um die Schieber (125, 126) an der vorbestimmten Stelle zu stoppen.

3. Airbag-Abdeckung nach Anspruch 2, bei der die Halteeinrichtung eine Verbindungseinrichtung aufweist, um das Paar von Schiebern (125, 126) miteinander zu verbinden.

4. Airbag-Abdeckung nach Anspruch 3, bei der die Verbindungseinrichtung ein von jedem Schieber (125; 126) getrenntes Element (180) ist, wobei die Verbindungseinrichtung eine erste Kopplungseinrichtung zur Kopplung mit dem ersten Schieber (125) und eine zweite Kopplungseinrichtung zur Kopplung mit dem zweiten Schieber (126) aufweist.

5. Airbag-Abdeckung nach Anspruch 1, bei der die Halteeinrichtung ein Verbindungsglied (115) aufweist, welches die Verschlußbänder (102) verbindet, wenn der Verschluß geschlossen ist.

6. Airbag-Abdeckung nach Anspruch 5, bei der das Verbindungsglied (115) eine Verbindung aufweist, die entkoppelt wird, um den Verschluß zu öffnen und wieder gekoppelt wird, um das Verbindungsglied (115) wieder zu bilden, nachdem es von der Kraft entkoppelt wurde.

7. Airbag-Abdeckung nach Anspruch 5 oder 6, bei der das Verbindungsglied (115) ein Matrizenelement und ein Patrizenelement aufweist, wobei das Matrizenelement mit dem Patrizenelement gekoppelt wird, um das Verbindungsglied (115) zu schließen.

8. Airbag-Abdeckung nach Anspruch 5 oder 6, bei der das Verbindungsglied (115) einen Vorsprung (150) aufweist, der sich von der Fläche der Verschlußbänder (102) weg erstreckt, wodurch das Verbindungsglied (115) entkoppelbar ist, wenn die Kraft auf den Vorsprung (150) aufgebracht wird.

9. Airbag-Abdeckung nach Anspruch 5, bei der das Verbindungsglied (115) ein zerbrechliches Element aufweist, welches zur Entkopplung des Verbindungsglieds brechbar ist.

10. Airbag-Abdeckung nach Anspruch 9, bei der das Verbindungsglied (115) einen verschmolzenen Bereich des Verschlußbands aufweist.

11. Airbag-Abdeckung nach Anspruch 9, bei der das Verbindungsglied (115) einen zusätzlichen Bandbereich aufweist, der an dem Verschlußband angeschmolzen ist.

12. Airbag-Abdeckung nach einem der vorhergehenden Ansprüche, bei dem die zur Freigabe oder zum Verrücken der Halteeinrichtung (115; 125; 126) erforderliche Kraft geringer als 400 N ist, wobei die Kraft auf einem Bereich des Verschlusses benachbart der Halteeinrichtung (115; 125; 126) in einer Richtung im wesentlichen senkrecht zu der Fläche des Verschlußbands (102) benachbart der Halteeinrichtung (115; 125; 126) aufgebracht wird.

13. Airbag-Abdeckung nach einem der vorhergehenden Ansprüche, bei der die zur Freigabe der Halteeinrichtung (115, 125; 126) erforderliche Kraft größer als 300 N ist, wobei die Kraft auf einem Bereich des Verschlusses benachbart der Halteeinrichtung (115; 125; 126) in einer Richtung im wesentlichen senkrecht zu der Fläche des Verschlußbands (102) benachbart der Halteeinrichtung (115; 125; 126) aufgebracht wird.

14. Airbag-Abdeckung nach einem der vorhergehenden Ansprüche, wobei die Abdeckung in der Form einer Abdeckung für einen Sitz (20) eines motorisierten Fahrzeugs vorliegt, wobei der Verschluß (100) an der Abdeckung (21) an der Öffnung (23) zum Schließen der Öffnung (23) befestigt ist.

15. Sitz (20) für ein motorisiertes Fahrzeug, wobei der Sitz einen Rahmen, eine nach einem der Ansprüche 1 bis 13 ausgebildete und an dem Rahmen angebrachte Abdeckung (21) und innerhalb des Rahmens einen Airbag (30) aufweist, wobei der Verschluß (100) an der Abdeckung an der Öffnung (23) zum Verschließen der Öffnung (23) bei Normalgebrauch und zum Öffnen unter dem Aufprall des Airbags, wenn der Airbag (30) aufgeblasen wird, um dem Airbag (30) das Passieren durch die Öffnung (23) zu erlauben, befestigt ist.

## Revendications

1. Elément de couverture de coussin gonflable comportant une ouverture (23) destinée à libérer à travers elle un coussin gonflable recouvert par l'élément de couverture lorsque le coussin gonflable se gonfle, l'ouverture étant fermée par une fermeture (100) comprenant des premier et deuxième rubans (102) de bande d'accrochage, comportant chacun une rangée d'éléments d'accouplement (104) montés sur lui de telle manière que lorsque la fermeture est fermée lesdits rubans (102) de bande d'accrochage sont reliés l'un à l'autre au moyen desdits éléments d'accouplement (104), des moyens de retenue (115 ; 125 ; 126) destinés à retenir ensemble lesdits rubans (102) à un emplacement prédéterminé, les moyens de retenue (115 ; 125 ; 126) étant libérés lorsqu'une force supérieure à une valeur prédéterminée est appliquée aux moyens de retenue (115 ; 125 ; 126) du fait de l'impact du coussin gonflable (30) lorsque le coussin gonflable se gonfle, afin de permettre aux éléments d'accouplement de se mettre hors de prise, **caractérisé en ce que**
chaque rangée d'éléments d'accouplement (104) comprend une première zone (106) et une deuxième zone (107) où les éléments d'accouplement (104) présents sur les premier et deuxième rubans (102) sont en prise lorsque la fermeture est fermée, les éléments d'accouplement (104) présents dans les première et deuxième zones (106 ; 107) se mettant hors de prise lorsque le coussin gonflable se gonfle et
un intervalle (110) est ménagé entre les première et deuxième zones (106 ; 107), les moyens de retenue (115 ; 125 ; 126) étant ménagés au niveau de l'intervalle (110) et les première et deuxième zones (106 ; 107) s'étendant sur des côtés respectifs opposés des moyens de retenue (115 ; 125 ; 126).

2. Elément de couverture de coussin gonflable selon la revendication 1 dans lequel ledit moyen de retenue comprend une paire de curseurs (125 ; 126), et dans lequel une butée d'arrêt (120) est ménagée en vue d'arrêter les curseurs (125 ; 126) à l'emplacement prédéterminé.

3. Elément de couverture de coussin gonflable selon la revendication 2 dans lequel ledit moyen de retenue comprend un moyen de liaison destiné à relier ensemble les curseurs (125 ; 126) de ladite paire de curseurs.

4. Elément de couverture de coussin gonflable selon la revendication 3 dans lequel ledit moyen de liaison est un élément (180) séparé par rapport à chacun des curseurs (125 ; 126), ledit moyen de liaison comportant un premier moyen de mise en prise destiné à une mise en prise avec le premier curseur (125) et un deuxième moyen de mise en prise destiné à une mise en prise avec le deuxième curseur (126).

5. Elément de couverture de coussin gonflable selon la revendication 1 dans lequel ledit moyen de retenue comporte un moyen de liaison (115) qui relie les rubans (102) de bande d'accrochage lorsque la fermeture est fermée.

6. Elément de couverture de coussin gonflable selon la revendication 5 dans lequel ledit moyen de liaison (115) comprend une connexion qui est mise hors de prise pour ouvrir la fermeture et qui est remise en prise pour restaurer le moyen de liaison (115) après avoir été mise hors de prise par ladite force.

7. Elément de couverture de coussin gonflable selon la revendication 5 ou selon la revendication 6 dans lequel ledit moyen de liaison (115) comprend un élément femelle et un élément mâle, ledit élément femelle étant mis en prise avec ledit élément mâle pour fermer le moyen de liaison (115).

8. Elément de couverture de coussin gonflable selon la revendication 5 ou selon la revendication 6 dans lequel ledit moyen de liaison (115) comprend une saillie (150) qui s'étend à l'écart de la surface des rubans (102) de bande d'accrochage, grâce à quoi ledit moyen de liaison (115) peut être mis hors de prise lorsque ladite force est appliquée sur ladite saillie (150).

9. Elément de couverture de coussin gonflable selon la revendication 5 dans lequel ledit moyen de liaison (115) comprend un élément cassable qui peut être brisé pour mettre le moyen de liaison hors de prise.

10. Elément de couverture de coussin gonflable selon la revendication 9 dans lequel ledit moyen de liaison (115) comprend une zone du ruban de bande d'accrochage qui a été fondue.

11. Elément de couverture de coussin gonflable selon la revendication 9 dans lequel ledit moyen de liaison (115) comprend une zone supplémentaire de ruban qui est fondue sur ledit ruban de bande d'accrochage.

12. Elément de couverture de coussin gonflable selon l'une quelconque des revendications qui précèdent dans lequel la force nécessaire pour libérer ou pour déplacer lesdits moyens de retenue (115 ; 125 ; 126) est inférieure à 400 N, ladite force étant appliquée sur une zone de la fermeture proche des moyens de retenue (115 ; 125 ; 126), dans une direction substantiellement perpendiculaire à la surface du ruban (102) de bande d'accrochage proche des moyens de retenue (115 ; 125 ; 126).

13. Elément de couverture de coussin gonflable selon l'une quelconque des revendications qui précèdent dans lequel la force nécessaire pour libérer les moyens de retenue (115 ; 125 ; 126) est supérieure à 300 N, ladite force étant appliquée sur une zone de la fermeture proche des moyens de retenue (115 ; 125 ; 126), dans une direction substantiellement perpendiculaire au plan du ruban (102) de bande d'accrochage proche des moyens de retenue (115 ; 125 ; 126).

14. Elément de couverture de coussin gonflable selon l'une quelconque des revendications qui précèdent, ledit élément de couverture de coussin gonflable se présentant sous la forme d'une housse de siège (20) de véhicule automobile, ladite fermeture (100) étant fixée à ladite housse (21) au niveau de ladite ouverture (23) en vue de fermer ladite ouverture (23).

15. Siège (20) pour véhicule automobile, ledit siège comportant un cadre, une housse (21), selon l'une quelconque des revendications 1 à 13, montée sur ledit cadre, un coussin gonflable (30) placé à l'intérieur dudit cadre, ladite fermeture (100) étant fixée à ladite housse au niveau de ladite ouverture (23) en vue de fermer ladite ouverture (23) en utilisation normale et de la faire s'ouvrir sous l'impact du coussin gonflable lorsque le coussin gonflable (30) se gonfle, afin de permettre que le coussin gonflable (30) passe par l'ouverture (23).
